# EUROPEAN PATENT APPLICATION

(11) **EP 2 174 982 A2**
(43) Date of publication of application: **14.04.2010**
(21) Application number: 08171645.8
(22) Date of filing: 07.10.2004
(51) Int. Cl.: C08L 39/06, C08L 53/00, C09J 133/06, C09J 153/00, A61L 24/00, A61L 15/24

(54) **Composition useful as a pressure sensitive adhesive and use of such a composition**

(30) Priority: 07.10.2003 DK 200301478
(62) Divisional of application: 04762896.1
(71) Applicant: Coloplast A/S, 3050 Humlebaek (DK)
(72) Inventor: Madsen, Niels Joergen, 3450, Alleroed (DK); Hoej, Carsten, 2720, Vanloese (DK)

(57) **Abstract**

A pressure sensitive adhesive composition comprising one or more hydrogel-forming hydrophilic homopolymers or heteropolymers and one or more amphiphilic block-copolymers comprising hydrophobic polymer blocks being incompatible and hydrophilic polymer blocks is useful, for example, in transdermal drug delivery systems and other medical, pharmaceutical and cosmetic products that adhere to the skin or other body surface.

## Description

### BACKGROUND OF THE INVENTION

### 1. Field of the Invention

The invention relates to a composition, in particular to a pressure sensitive adhesive composition, suitable for application to human or animal skin, to a method for preparing such compositions and the use of such compositions for the preparation of a wound dressing or a adhesive wafer for an ostomy appliance or the use of the compositions for securing of and sealing around ostomy bandages, for securing wound dressings, for securing of devices for collecting urine, wound-drainage bandages, orthoses and prostheses and for protection skin areas and parts of the body against pressure, impacts and friction and to wound dressings or ostomy appliances comprising such compositions.

In particular, the invention relates to hydrophilic pressure sensitive adhesives or hydrogel adhesive having an optimised degree of adhesion to moist and wet skin and mucous membranes and furthermore having an optimised cohesiveness during and following hydration.

Furthermore, the invention relates to the use of the compositions of the invention for coating of medical appliances, such as catheters to be inserted into a body cavity. Other embodiments of the invention are foams prepared from the compositions of the invention and electroconductive hydrogels based on the composition of the invention.

### 2. Description of the Related Art

Pressure sensitive adhesives for fixation of medical devices on human skin, for wound treatment, for drug delivery and for a variety of medical, cosmetic and industrial uses comprising hydrophilic elements, are known

Thus, US Patent No. 6,576,712 Feldstein et al. discloses a hydrophilic pressure sensitive adhesive composition comprising a hydrophilic polymer and a complementary short-chain plasticizing agent such as polyethylene glycol wherein the hydrophilic polymer and plasticizing agent are capable of hydrogen bonding or electrostatic bonding to each other.

A commonly used pressure sensitive adhesive composition for application to skin comprises an adhesive elastomeric matrix having water-absorbing, swelling particles, the so-called hydrocolloids, dispersed therein.

WO 98/48858 discloses a pressure sensitive adhesive composition suitable for application to human or animal skin comprising a conjugated diene polymer, a polyvinyl pyrrolidone polymer or a polyvinyl pyrrolidone vinylacetate copolymer, optionally one or more hydrocolloids and optionally a physically cross-linked elastomer selected from block-copolymers comprising styrene and one or more butadienes .

GB 2 064 556 describes compositions capable of forming a hydrogel without being dissolved and the use thereof for burn- and wound dressings, coatings for catheters and surgical sutures, soft contact lenses, implants for delivery of medicaments at controlled rates, and other articles coming into intimate contact with body tissues or cavities. These hydrogel forming compositions comprises a water soluble poly(vinylpyrrolidone) and a water insoluble copolymer formed from hydrophobic water-insoluble ethylenically unsaturated monomer, an ethylenically unsaturated monomer containing an acid group and optionally a hydrophilic ethylenically unsaturated monomer free from acidic groups.

According to GB 2 064 556, the compositions described therein behave as a physical mixture and possess thermoplastic properties. Microphase domains of water-insoluble material dispersed in the continous phase of water soluble poly(vinyl pyrrolidone) has been observed in the electron microscope. In order for the composition to have satisfactory mechanical properties in hydrated from the size of the micro domains should not be more than 4,000 A and should preferably be below 1000 A.

GB 2 086 400 describes compositions of the same type as GB 2 064 556.

The compositions according to the invention differs from the composition described in GB 2 064 556 and GB 2086 400, in that it contains an amphiphilic block copolymer with distinct hydrophobic and hydrophilic polymer blocks instead of a copolymer with an random sequence of monomers.

According to the present invention a hydrogel forming composition, e.g. a pressure sensitive adhesive, with high water absorption, improved integrity (improved cohesive properties) and mechanical strength has been provided.

A further advantage of the compositions of the invention is that they are thermoplastic.

The compositions are also useful, for example, in iontophoretic systems, biomedical electrode fabrication, wound healing, skin care, transdermal drug delivery systems and other medical, pharmaceutical and cosmetic products that adhere to the skin or other body surfaces.

The adhesive composition of the invention absorbs and transports moisture very efficiently and quickly absorbs the moisture present on wet skin. The adhesive composition of the invention is therefore particularly useful on wet skin.

Another advantage obtained by the pressure sensitive adhesive compositions of the invention is improved adhesion to wet or moist skin or tissue as well as mucosa, which means that these new adhesive formulations have increased wet tack properties compared to ordinary hydrocolloid adhesive or acrylic adhesive.

The adhesive compositions of the invention may also be used for securing ostomy appliances to the skin and for sealing around an ostomy, for securing wound dressings or wound drainage bandages to the skin, especially for securing devices for collecting urine to the skin, or for securing orthoses or prostheses to the skin. The ostomy appliances or wound dressings may be any such product known per se and may be prepared in a manner analogous to the preparation of similar products with conventional adhesive compositions.

### SUMMARY OF THE INVENTION

The present invention provides a composition comprising one or more hydrogel-forming hydrophilic homopolymers or heteropolymers and one or more amphiphilic block-copolymers comprising hydrophobic polymer blocks being incompatible and hydrophilic polymer blocks being compatible with the hydrogel-forming hydrophilic homopolymers or heteropolymers.

In a particular embodiment of the invention, the composition is a pressure sensitive adhesive.

### Detailed Description of the Present Invention

The amphiphilic block-copolymer containing a block being compatible with the hydrogel-forming hydrophilic homopolymers or heteropolymers and a block being incompatible with the same will provide a degree of physical cross-linking of the hydrogel-forming hydrophilic homopolymers or heteropolymers. When using diblock amphiphilic copolymers the physical association of hydrophobic end blocks in separate domains will provide a considerable increase and control of the viscosity of the composition and when using tri-block amphiphilic copolymers the physical cross-linking will provide a pronounced cohesion effectively stabilising the hydrogel and enabling the removal thereof essentially without leaving remains on the skin.

As used herein physical cross-links mean reversible cross-links based on segregation of domains caused by secondary interactions between the polymer chains. These secondary interactions include hydrogen bonding and ionic bonding, but not covalent bonding.

Unlike covalent cross-links, physical cross-links may be broken when heated and established again upon cooling. This allows the material to be processed, and recycled.

As used herein compatible means the ability for two or more molecules to homogenously associate in a single phase.

As used herein incompatible means the inability of two or more molecules to homogenously associate in a single phase.

The hydrophilic homopolymer or heteropolymer useful in the composition according to the invention, should suitably be a hydrophilic homopolymer or heteropolymer which provide a very effective wet tack on human skin rendering it suitable for application on humid skin or exuding wounds, or on moist skin around a stoma. Either the hydrogel forming homopolymer of heteropolymer has in itself adhesive properties (intrinsic adhesive properties) or the hydrogel forming homopolymer of heteropolymer is compounded with a tackyfier or plastiziser which provide or improve the adhesive properties of the composition.

The hydrophilic homopolymer or heteropolymer is suitably a cellulose derivative, a polysaccharide, polyvinyl-pyrrolidone, polyvinyl alcohol, polycarboxylic acid polyacrylic acid, poly (methyl vinyl ether/ maleic anhydride), poly (meth)acrylic acid, polyethylenglycols (PEG), polyamides, polyacrylic amides and derivatives or blends of these polymers, or hydrophilic copolymers prepared from the same type of monomers as the above mentioned polymers.

Preferred, the hydrophilic homopolymer or heteropolymer is a poly-vinylpyrrolidone polymer or a copolymer containing vinylpyrrolidone.

PVP types useful as hydrogel forming hydrophilic homopolymers or heteromers are:

| PVP | Molecular weight* | Supplier |
|---|---|---|
| ViviPrint 540 | cross-linked | ISP |
| PVP K-90 | 1.000.000-1.500.000 | ISP and BASF |
| PVP K-30 | 45.000-60.000 | ISP and BASF |
| PVP K-25 | 28.000-34.000 | ISP and BASF |
| PVP K-15 | 7.000-11.000 | ISP and BASF |
| PVP K-12 | 2.000-3.000 | BASF |
| PVPNA S 630copolymer | | ISP and BASF |

| | | |
|---|---|---|
| *The molecular weights (Mw) of the PVP polymers was given by the manufacturer and is determined by light scattering. | | |

Other preferred hydrophilic homo- or heteropolymers are acrylic acids or copolymers containing acrylic acid, such as hydrophilic copolymers of a poly (methyl vinyl ether/ maleic anhydride) or poly (methyl vinyl ether/ maleic-acid).

The ability of polymers based on acids, such as acrylic acid, to absorb water and transport water (water and vapour permeability) is highly pH dependent. Such polymers has to be neutralised with a base before use in order to obtain a polymer capable of absorbing and transporting sufficient amounts of water. Suitable, the pH should be around 7 in order to achieve the desired properties with such polymers.

Specific hydrogel forming hydrophilic polymers are the polymers selected from the group consisting of Luvicross® (ia crosslinked and thus insoluble vinylpyrrolidone homopolymer), Luviskol® VA 37 E, Luviskol® VA 55 IVPNA Copolymer, Luviskol® VA 64 Powder and Luviskol® VA 73 E (all copolymers of vinylpyrrolidone and vinylacetate), Luvitec® VPI 55 K 72 W a vinylpyrrolidone/vinylimidazole and Luvitec® VPC 55 K 65 W (a vinylpyrrolidone/vinylcaprolactam copolymer), Luvicap® EG (a Polyvinylcaprolactam polymer from BASF), Luviset P.U.R. (a neutralized anionic polyurethane polymer), Luviquat Hold, Polyquatemium-46 (a copolymer of vinylcaprolactam (VCap), vinylpyrrolidone (VP) and quatemized vinylimidazole), Luviquat PQ 11 PN, Polyquatemium-11 (a quatemized copolymer of vinylpyrrolidone (VP) and dimethylaminoethylmethacrylate (DMAEMA), Luviquat UltraCare, Polyquaternium-44. Luviquat Care, Polyquaternium-44. Luviquat HM 552 Polyquatemium-16, Luviquat Style Polyquatemium-16, Luviquat FC 370 Polyquatemium-16, Luviquat FC 550 Polyquaternium-16 and Luviquat Excellence Polyquatemium-16 (all copolymers of vinylpyrrolidone (VP) and quaternized vinylimidazole), Polyquatemium-16 and Polyquaternium-44 (copolymers of vinylpyrrolidone (VP) and quatemized vinylimidazole (QVI), or Pluracare® F 68 NF, Poloxamer 188. Pluracare® F 87 NF, Poloxamer 237. Pluracare® F 108 NF Poloxamer 338. Pluracare® F 127 NF. Poloxamer 407, Pluracare® L 44 NF and Poloxamer 124 (all block copolymers and synthetic copolymers of ethylene oxide and propylene oxide) and polymethylvinylether

A preferred group of hydrophilic homo- or heteropolymers are polymers having intrinsic adhesive properties such as for example, polymethylvinylethers.

In other cases it is necessary to add a tackyfier and /or a plastiziser in order to achieve the desired adhesive properties.

### Suitable tackifiers are:

Eastman AQ1045 with a 4500cp melt viscosity, Eastman AQ1350 with a 35000cP melt viscosity, Eastman AQ1950 with a 95.000cP melt viscosity, Eastman AQ14000 with a 400.000cp melt viscosity (all from Eastman Chemical Company), Nevex 100 aromatic (Neville Chemical Co. Pittsburgh, PA), Benzoflex 352 Solid Benzoate Ester Plasticizer, Benzoflex 9-88 Liquid Plasticizer ( both from Velsicol Chemical Corporation), C5-C9 tackyfier resin (Arkon P 90, Arakawa), C5 resins such a EASTOTAC™ H-100W, hydrogenated C9 hydrocarbon Resins such as REGALITE™ R1125, partial hydrogenated C9 aromatic resins such as REGALITE™ S5100 and REGALREZ™ 6108), pure monomer styrene-based resins such as REGALREZ™ 1094, Cellolyn 21-E Synthetic Resin, Foral 85-E Ester of Hydrogenated Rosin, Foralyn 110 Ester of Hydrogenated Rosin, Pentalyn H-E Ester of Hydrogenated Rosin. Permalyn 2085 Synthetic Resin, Staybelite Ester 3-E Ester of Hydrogenated Rosin Staybelite Resin-E Partially Hydrogenated Rosin (all from Eastman Chemical Company), Lutonal® A 25 Lutonal® A 50, 50% in Ethanol and Lutonal® I 60, 80% In Mineral Spirits. Lutonal M40 (all from BASF), Vistanex™ LM or OPPANOL® (also from BASF),

For some applications it is preferred that the adhesive composition of the invention comprises a plasticizer for the hydrophilic homopolymer or heteropolymer in order to ensure optimum elastic and plastic moduli for the intended use as soft skin or mucosa adhesives. This is especially the case when the hydrophilic homopolymer or heteropolymer is a polymer of a relatively high degree of polymerisation.

Suitable, the hydrophilic plasticizers for use in the adhesive compositions of the invention are selected from polyethylene glycol (e.g. PEG 300, PEG 400), propylene glycol, dipropylene glycol, glycerol, glycerol diacetate (diacetin), glycerol triacetate (triacetin), triethyl citrate (Citrofol AI), acetyl triethyl citrate (Citrofol AII) and water.

When using a plasticizer in the adhesive compositions of the invention, it is preferably selected from the group consisting of polyethylene glycols, such as PEG 400, and water.

When preparing the adhesive composition of the invention, the plasticizer is suitably added in an amount of from 20 to 50% by weight of the total weight of the desired composition, i,e. before use.

According to the invention, the amphiphilic polymer contains hydrophobic blocks incompatible with the hydrogel-forming hydrophilic homopolymers or heteropolymers. When preparing the adhesive composition of the invention, the amphiphilic polymer is suitably added in an amount of from 10 to 90%, preferably 10-80 % or most preferred 10-90 % by weight of the total weight of the desired composition, i.e. before use.

Amphiphilic block polymers consist of a non-polar polymeric chain covalently linked to a polar polymeric chain. More particularly the polar chain end of the polymer must be water-soluble or water swellable and take up at least a content of 300 % w/w, preferably at least 500 % w/w of water based on dry matter, if taken alone. The non-polar chain preferably does not take up more than 10 % w/w of water based on dry matter when submersed in water.

Suitably, monomer units polymerised to form the hydrophobic blocks are identical or essentially identical and the monomer units polymerised to form the in the hydrophilic block(s) are identical or essentially identical, or the monomer units polymerised to form the in the hydrophilic block(s) are different monomer units (e.g. as a copolymer).

In a particular embodiment, the monomer units polymerised to form the in the hydrophobic blocks are identical or essentially identical and the monomer units polymerised to form the hydrophilic block(s) are different monomer units (e.g. as in a copolymer).

In another embodiment, the hydrophilic block(s) is prepared from identical monomer units or essentially identical monomer units and the hydrophobic block(s) is prepared from identical monomer units or essentially identical monomer units, or the hydrophobic block(s) is prepared from different monomer units. In a particular embodiment the hydrophilic block(s) is prepared from identical monomer units or essentially identical monomer units and the hydrophobic block(s) is prepared from different monomer units.

According to still another embodiment, the hydrophobic block(s) is prepared from identical monomer units or essentially identical monomer units and the hydrophilic block(s) is prepared from identical monomer units or essentially identical monomer units.

The amphiphilic block co-polymers are made from two or more blocks of hydrophilic monomers or hydrophobic monomers. The polymers may for instance be a diblock having a structure AB, where A is a hydrophobic block and B is a hydrophilic block or a triblock having a linear structure ABA where A is a hydrophobic block and B is a hydrophilic block, or alternatively have the form of a multi block or three or multi arm star-shaped copolymer structure, containing A and B blocks. Suitably, the amphiphilic block co-polymer is a diblock AB or a triblock ABA, mpst preferred the amphiphilic block co-polymer is a triblock copolymer ABA.

The incorporation of amphiphilic block copolymers having long hydrophobic end blocks into the adhesive composition of the invention improves the cohesion dramatically compared to the incorporation of conventionally used associative thickeners.

Due to the physical cross-linking, the amphiphilic block copolymers maintain a high cohesion in the adhesive during and following hydration and water absorption.

The hydrophobic block(s) of the amphiphilic block-copolymer will constitute separate physically cross-linked domains being incompatible with the continuous hydrophilic phase. Thus, in an adhesive containing a styrene-acrylic acid or a styrene-vinylpyrrolidone block copolymer the hydrophobic styrene blocks is incompatible with the domains formed by the hydrogel forming hydrophilic homopolymer or heteropolymer, fx acrylic acid or polyvinylpyrrolidone. The hydrophobic polystyrene blocks form discrete domains, which act as physically cross-links.

Hydrophobic monomers for preparing the A block are suitably monovinyl aromatic monomers which typically contain from about 8 to about 18 carbon atoms such as styrene, alpha-methylstyrene, vinyltoluene, vinylpyridine, ethylstyrene, t-butylstyrene, isopropylstyrene, dimethylstyrene, and other alkylated styrenes, or the hydrophobiv monomers for use in block A are (meth)-acrylic esters, or vinyl esters.

Altematively, the A block may be prepared from ethylenically unsaturated monomers chosen from butadiene, chloroprene, (meth)acrylic esters, vinyl esters such as vinyl acetate, vinyl versatate and vinyl propionate; or vinyl halides such as vinyl chloride, and vinyl nitriles.

"(Meth)acrylic esters" is used in the present context to designate esters of acrylic acid and of methacrylic acid with optionally halogenated, e.g. chlorinated or fluorinated, C₁ -C₃₀ straight or branched alcohols, preferably C₁ -C₁₈ alcohols. Examples of such esters are methyl acrylate, ethyl acrylate, propyl acrylate, n-butyl acrylate, isobutyl acrylate, 2-ethylhexyl acrylate, lauryl acrylate, tert.butyl acrylate, methyl methacrylate, ethyl methacrylate, n-butyl methacrylate and isobutyl methacrylate.

Suitable vinyl nitriles are those having from 3 to 12 carbon atoms, such as, in particular, acrylonitrile and methacrylonitrile.

It also considered an embodiment of the present invention to replace styrene completely or partly by derivatives thereof such as alpha-methylstyrene or vinyltoluene.

Thus, the hydrophobic part A of the amphiphilic block copolymer may suitably be a polystyrene, a poly alpha-olefin such as polyethylene, polypropylene, poly-1-butene or polyisobutylene, a poly acrylate, a polyvinylether, a polyacetate, a polysiloxane, a hydrophobic polyester or similar polymers conventionally used in pressure sensitive adhesive formulations.

For use in accordance with the present invention it is suitable that the hydrophobic block(s) of the amphiphilic polymer comprises polymerised styrene, i.e the block consists essentially of polymerised styrene or the block consists of a copolymerised styrene monomer with other hydrophobic monomers.

In a suitable alternative embodiment of the invention the hydrophobic block(s) of the amphiphilic polymer comprises polymerised acrylic acid ester monomers, i.e the block(s) consists essentially of polymerised acrylic acid ester or the block(s) consists of copolymerised acrylic acid ester monomers and other hydrophobic monomers.

In a further embodiment of the invention the hydrophobic block(s) of the amphiphilic polymer comprises hydrophobic blocks of a polymerised vinylic unsaturated aliphatic hydrocarbon monomer comprising from 1 to 6 carbon atoms. Preferred and commercially readily available are polymerised vinylic unsaturated hydrocarbon monomers, comprising 4 carbon atoms, polybutylene and polyisobutylene being most preferred.

The most preferred hydrophobic blocks are blocks consisting essentially of polystyrene, polymethylmethacrylate, polybutylacrylate , or poty-2-ethylhexylacrylate.

Alternatively, the amphiphilic block copolymer comprises different hydrophobic blocks A , e.g a block of styrene and a block of a hydrophobic polyacrylic acid ester.

A hydrophobic plastiziser may be added to the adhesive composition of the invention.

Suitable hydrophobic plasticizers for use in the adhesive compositions of the invention are e.g. camphor, castor oil, dibutyl phthalate, dibutyl sebacate, dioctyl adipate (DOA), dioctyl adipate (DOP), acetyl tributyl citrate (Citrofol BII), santicizer 141, Santicizer 148, Santicizer 261, Sebacic acid, and Tributyl citrate (Citrofol BI).

The hydrophilic blocks of the amphiphilic block copolymer which is compatible with the hydrogel matrix of a given adhesive may be any type of polymer having the same chemical structure as the hydrophilic matrix of the adhesive, or having the same type of chemical structure (PVP, acrylate), or having physical properties that allow the block to coexist in the same phase as the hydrogel forming hydrophilic homo- or heteropolymer matrix.

The hydrogel forming hydrophilic polymer (e.g. polyacrylic acid or polyvinyl-pyrrolidone) domains in such adhesives typically contain a plasticizer and may furthermore, if desired, contain tackifying resins and will generally be softer than the more crystal-like hydrophobic (fx polystyrene) domains.

Due to the phase separation and formation of a physically cross-linked polymer system by the hydrophobic blocks of the amphiphilic block copolymer, comprising styrene or other hydrophobic blocks, and their covalent bonding to blocks of one or more hydrophilic blocks in the amphiphilic block copolymer, which are capable of forming hydrogen bonding or electrostatic bonding to the hydrogel-forming hydrophilic homopolymers or heteropolymers, i.e. the matrix forming polymers, the risk of exudation separation is reduced or even eliminated.

Thus, in one embodiment of the invention in which the hydrogel-forming hydrophilic homopolymer or heteropolymer is a polyvinylpyrrolidone, the hydrophilic block of the amphiphilic polymer must be compatible with polypolyvinylpyrrolidone or optionally with polyvinylpyrrolidone compounded with a plasticizer.

In another embodiment of the invention in which the hydrogel-forming hydrophilic homopolymer or heteropolymer is a carboxylic acid or acrylic acid polymer, the hydrophilic block of the amphiphilic polymer must be compatible with the hydrogel phase comprising the polycarboxylic or polyacrylic acid polymer.

The hydrophilic part of the amphiphilic block copolymer (B block) may be any type of polymer compatible with the hydrogel-forming hydrophilic homopolymers or heteropolymers and incompatible with the hydrophobic block of the amphiphilic block co-polymer, that will be able to absorb significant amounts of water.

Hydrophilic monomers useful for preparing the B block are for example ethylenically unsaturated monocarboxylic and dicarboxylic acids, such as acrylic acid, methacrylic acid, itaconic acid, maleic acid and fumaric acid; and monoalkyl esters of dicarboxylic acids of the type mentioned above with alkanols, preferably alkanols having from 1 to 4 carbon atoms and their N-substituted derivatives (amides), amides of unsaturated carboxylic acids, such as acrylamide, methacrylamide, N-methoxyacrylamide or methacrylamide, and N-alkylacrylamides; ethylenic monomers containing a sulphonic acid group and ammonium or alkali metal salts thereof, for example vinylsulphonic acid, vinylbenzenesulphonic acid, alpha-acrylamidomethylpropanesulphonic acid and 2-sulphoethylene methacrylate; amides of vinylamine, especially vinylformamide or vinylacetamide; and unsaturated ethylenic monomers containing a secondary, tertiary or quaternary amino group, or a heterocyclic group containing nitrogen, such as, for example, vinylpyridines, vinylimidazole, aminoalkyl (meth)acrylates and aminoalkyl (meth)acrylamides such as dimethylaminoethyl acrylate or methacrylate, di-tert.butylaminoethyl acrylate or methacrylate and dimethylaminoacrylamide or dimethylaminomethacrylamide. It is also possible to use zwitterionic monomers such as, for example, sulphopropyl(dimethyl)-aminopropyl acrylate.

Suitable, hydrophilic polymer blocks in the amphiphilic block copolymers for use in accordance with the present invention are PEG (polyethylene glycol), poly ethylene oxide, PVP (polyvinyl pyrrolidone), polyacrylic acid, salts of polyacrylic acid, salts of polymers of composed of acids such as maleic acid, polyvinyl alcohol, hydrophilic polyurethanes, poly hydroxyethylmethacrylate (HEMA), polyethyleneglycol(meth)acrylate, polyethoxypolyethyleneglycol(meth)acrylate, polymethoxyethyl(meth)acrylate, polyethoxy(meth) acrylate, poly 2-dimethylaminoethyl(meth)acrylate (DMAEMA) and poly 3-dimethylaminopropylmethacrylamid (DMAPMA), carbohydrates or gelatins.

The hydrophilic block(s) in the amphiphilic block copolymers may also be prepared from different monomers, or oligomers, for example the monomer or oligomers used for the preparation of the above mentioned hydrophilic polymer blocks, or monomers selected from acrylic acid, maleic acid, hydroxyethylmethacrylate (HEMA), vinylpyrrolidone (NVP), polyethyleneglycol(meth)acrylate, ethoxypolyethyleneglycol(meth)acrylate, methoxyethyl(meth)acrylate, ethoxy(meth) acrylate, 2-dimethylaminoethyl(meth)acrylate (DMAEMA) and 3-dimethylaminopropylmethacrylamid (DMAPMA), in order to impart desired properties (e.g Tg and modulus) into the polymer. Suitably, the hydrophilic block(s) may be a copolymer of neutralised acrylic acid, such as sodium acrylate and hydroxyethylmethacrylate (HEMA), vinylpyrrolidone (NVP), polyethyleneglycol(meth)acrylate, ethoxypolyethyleneglycol(meth)acrylate, methoxyethyl(meth)acrylate, ethoxy(meth) acrylate, 2-dimethylaminoethyl(meth)acrylate (DMAEMA) or 3-dimethylaminopropylmethacrylamid (DMAPMA). Thus, the hydrophilic block may also be a random copolymer.

As used herein (meth)acrylate means acrylate and methacrylate.

It is also possible to have a number of hydrophobic monomers, present in the hydrophilic blocks, e.g. in order to impart desired properties into the polymer, as long as the presence thereof provide a hydrophilic block that will be able to absorb significant amounts of water and which is compatible with the hydrophilic homo- or heteropolymer and incompatible with the hydrophobic block(s) of the amphiphilic block copolymer(s).

Likewise, it is possible to have a number of hydrophilic monomers, present in the hydrophobic block(s) as long as the presence thereof provide a block which is incompatible with the with the hydrophilic homo- or heteropolymer and compatible with the hydrophobic block(s) of the amphiphilic block copolymer(s).

The hydrophilic block preferably has a minimum molecular weight of about 500 in order to be able to form separate hydrophilic domains in the adhesive composition.

Suitably, the hydrophilic block(s) of the amphiphilic block copolymer has a molecular weight of at least 1000, preferably between 1000 and 300.000, more preferred between 50.000 and 300.000.

Preferably the molecular weight is higher than 1000 in case of end blocks and 5000 in case of midblocks

The size or molecular weight of the hydrophobic block A does not appear to be a limiting factor for the use of an amphiphilic block copolymer for the purpose of the present invention, as long as the hydrophobic block is sufficiently large to form a physical separation of the hydrophobic and hydrophilic phases in the adhesive composition, i.e. hydrophobic domains or phases are formed.

Generally, the hydrophobic block(s) of the amphiphilic block copolymer has a molecular weight of at least 1000, preferably between 1000 and 500.000, more preferred between 1000 and 300.000, more preferred between 1000 and 100. 000, or most preferred between 1000 and 50.000.

Preferably, hard hydrophilic blocks (i.e. polymers with low Tg) have a smaller size than softer hydrophilic blocks (i.e. blocks with a higher Tg). For hard hydrophobic blocks, such as styrene blocks, the molecular weight of the block is typically between 10.000 and 20.000 and for soft blocks, such as butylacrylate, it is suitable between 50.000 and 100.000.

A person skilled in the art will be able to select the appropriate size for the hydrophobic block(s) in view of the size of the hydrophilic block (s) and vice versa.

Preferred amphiphilic block copolymers to be used in accordance with the present invention are such wherein the A domain is a thermoplastic polymer end block of a mono vinyl aromatic homo polymer, preferably one having at least two different molecular weight end blocks in the copolymer (e.g., one A block of about 1000 to about 50,000 number average molecular weight and a B block of about 1000 to about 500,000 number average molecular weight), where the B domain is a hydrophilic end block polymer or the mid polymer block in case of a triblock copolymer.

Preferred the amphiphilic block copolymer is a triblock copolymer ABA having the hydrophilic block B as the midblock.

In yet another preferred embodiment of the present invention the amphiphilic polymer is an amphiphilic polyurethane block copolymer. Suitable amphiphilic polyurethanes for the purpose of the present invention are ESTANE T5410 from NOVEON (B.F.Goodrich), Tecophilic HP93A100, Tecogel 500 or 2000 from Thermedics Polymer Products, or HydroMed D640 from CardioTech International Inc.

Amphiphilic or hydrophilic block copolymers for use in accordance with the present invention may be functionalised for further reactions like graft copolymerisation, cross-linking or for further polymerisation by inclusion of suitable functional groups.

The functional groups may be attached to the ends of the main chains or as side chains. The functional groups may e.g. be unsaturated vinyl groups containing double bonds for further polymerisation. The functional groups may furthermore be photo initiators attached to the block copolymers for UV-polymerisation or for cross-linking. The functional groups may be hydroxyl, primary or secondary amine groups for further reactions with isocyanate for the formation of polyurethane based block copolymers or for cross-linking with isocyanate.

Suitable amphiphilic block copolymers for the purpose of the present invention are a poly(styrene-b-acrylic acid-b-styrene) (P3000-SAAS with Mn 2000-65000-2000), poly(methyl methacrylate-b-methacrylic acid-b-methyl methacrylate) (P1483-MMAMAAMMA) and poly(styrene-b-ethylene oxide-b-styrene) (P2525-SEOS with Mn at 9500-48000-9500) all available from Polymer Source 124 Avro Street, Montreal, Quebec H9P 2X8. Canada as well as commercially available amphiphilic block copolymers from Rhodia and Atofina.

Amphiphilic block copolymers useful according to the invention may be prepared by a number of methods, such as free-radical polymerisation controlled by xantates, dithioesters, dithiocarbamates, iniferters, iodine degenerative transfer, tetraphenylethane derivatives, or organocobalt complexes, or by polymerisation using nitroxide precursors, atom transfer free-radical polymerisation (ATRP) and group transfer polymerisation.

These methods with references are mentioned in US Patent publication 2004/0030030.

The compositions according to the invention may also comprise a cohesion-promoting component such as polyacrylic acid or polycarboxylic acid, acrylic acid copolymers or associative thickeners.

Associative thickeners are polymers that are based on water-soluble polymers. They can be acrylate polymers, cellulose ethers or, polyethylene-glycol. They are capped with water-insoluble hydrophobic groups like fatty alcohols, for example. In water solution or in emulsion, these polymers form a network that increases the viscosity. The water-soluble backbone polymer is dissolved in water. The hydrophobic caps are adsorbed onto the hydrophobic emulsion polymer particles, or they form micelle structures with hydrophobes from other polymers. As each associative thickener polymer contains at least two hydrophobic caps, the result is a three-dimensional network within the emulsion. This increases the viscosity. Mainly the high- and mid-shear viscosity is affected.

Thickeners are suitably STABILEZE® 06 & QM, GANTREZ®, polymethyl vinyl ether/maleic anhydride copolymers from ISP, Aculyn™ 28 from Rohm and Haas. NEXTON® or Natrosol® Plus CS both hydrophobically modified hydroxyethylcelluloses from HERCULES, or Carbopol homopolymers and copolymers such as Noveon AA1 and Pemulen TR 2 from Noveon.

Such cohesion promoting component is suitably added during preparation of the adhesive composition in an amount of up to 15% by weight of the total weight of the composition prepared, i.e before use.

Still further, the compositions according to the invention may also comprise conventionally used extenders or fillers such as polysaccharides like CMC, antioxidants, fibres, salts, clay, buffers, or pigments in an amount up to 30% of the total weight of the composition prepared.

The appropriate amount of the different ingredients in the adhesive composition of the invention is best expressed by listing the amounts of the various ingredients used for the preparation of the adhesive. The amounts will more or less correspond to the amounts in the final adhesive composition although a minor variation in the amount of water (before use) may cause small or minor changes the percentages of the various ingredients in the composition. Thus, the adhesive of the invention may suitably be prepared from:
At least 5 % w/w of a hydrogel forming hydrophilic homopolymer or heteropolymer;
10-90 % w/w, preferably 10-50 % of an amphiphilic block copolymer;
0-20 % w/w of a tackifier resin
0-30 % w/w of a plastiziser for the hydrophobic phase
0-50 % w/w of a plastiziser for the hydrophilic phase.

For the preparation of the composition of the invention, the hydrogel-forming hydrophilic homopolymers or heteropolymers are suitable added in an amount from 5 to 80%, preferably 10-80 % by weight of the total weight of the desired composition, i.e. before use.
The compositions according to the invention may be processed in analogy with conventional methods for preparing thermoplastic adhesives. The preferred method will be by hot melt processes. This includes blending in hot melt mixers like Z-blade mixers, single or double barrel extruders, planet-mixers or equivalent equipment followed by a coating or moulding step to given substrates or release liners. Alternatively the composition may be cast form a solution at suitable release liners or backings and added any net or non-woven for reinforcing or improving handling. Still further the composition may be foamed, coated or formed into any desired thickness or shape. A general outline of preferred mixing and coating technologies that may apply for the invention can be found in D Satas "Handbook of Pressure Sensitive Adhesive Technology" 3rd edition 1989 (Satas & Associates) chapter 38: Coating Equipment.

Preferred methods of mixing and production of hydrophilic pressure sensitive adhesive are batch mixing in high shear Z-blade type of mixers, continuously in single or double screw extruders or by casting from solvent solutions.

The composition of the invention may be foamed and foamed adhesives are especially interesting as they may provide improved moisture handling properties, improved adhesion to flexible and/or uneven substrates and potentially improved skin compatibility.

The foamed compositions may be characterised by porosity, stability, open/closed or mixed cell structure and cell size distribution. The higher the porosity, the softer and more flexible a foam is produced with minimal consumption of polymer composition. It is preferred that the porosity is between 10 and 80%, and more preferred between 40 and 70 %. Open cells will yield a higher moisture absorption and transmission rate. Closed cells will provide the best physical stability. Foamed adhesive with small sized cells may be preferred.

The foamed compositions may be produced by conventional methods, such as mechanical introduction and dispersion of a suitable expanding moiety, e.g. compressed air, nitrogen, carbon dioxide, argon or other gasses or low boiling point liquids. Suitable equipment may include the "FoamMelt ®" and "FoamMix ®" machines available from the Nordson Corporation.

The foamed compositions may alternatively be produced by compounding the composition with a suitable chemical blowing agent, which may generate gas bubbles by a variety of mechanisms. These mechanisms include, but are not limited to chemical reaction, thermal decomposition or chemical degradation, volatilisation of low boiling materials, expansion of gas filled materials or by a combination of these methods.

The term chemical blowing agent is used herein to cover the use of single or multiple component chemicals in a mixture or paste. Suitable chemical blowing agents include the carbonates of alkali metals, such as ammonium carbonate, ammonium bicarbonate, sodium carbonate, sodium bicarbonate, and calcium carbonate. Improved gas generation may be obtained by preparing a mixture of carbonates of alkali metals and various organic acids. Other suitable blowing agents includes expanding spheres such as "Expancel ®" available from Akzo Nobel.

The foamed composition of the invention may be coated on to a substrate or otherwise shaped by a wide number of possible processes including reverse roll coating, slot die coating and different moulding techniques such as injection and vacuum moulding. The foamed composition may by further shaped by e.g. cutting or bevelling and it may be laminated onto other materials. The foamed composition may be coated into thin layers, as well as it may be moulded into three-dimensional structures.

Wound dressings or other dressings comprising the adhesive composition of the invention may be prepared by coating or laminating the adhesive composition to a film, for example a polyurethane film or any other film material which is conventionally used for dressings.

Likewise, the composition of the invention may be coated or laminated on any medical appliance or part of a medical appliance or device to be attached to the skin of a living being.

Compositions according to the invention, including the adhesive composition of the invention, will, when subjected to an excess amount of water, for example by immersing the composition into water and leaving it there, in some cases for an extended period of time, gradually loose its adhesive properties and instead achieve a highly slippery surface as the content of water in the composition increases. Even after prolonged immersion in water or aqueous solution, the composition of the invention does not fall apart, but forms a hydrogel held together by a strong cohesion.

This is not a problem during use of the composition of the invention as an adhesive composition, for example for a wound dressing or sealing around a stoma, where the amount of fluid is considerably lower.

A composition of the invention may, when it contains so much water or aqueous solution that the composition has a slippery surface may be used as a slippery hydrophilic coating for medical appliance to be inserted into natural or artificial body cavities of a living being.

Thus, according to further embodiment, the invention relates to a medical appliance, such as for example a surgical suture, a cathether or a guidewire, for introduction into a natural or artificial body cavity of a living being, where at least the part of said appliance to be inserted into said body cavity comprises a composition according to the invention.

On one embodiment, the medical appliance carries the composition of the invention as a coating at least on its outer surface.

The coating may be applied to the medical appliance using techniques known for coating of e.g. catheters. Injection moulding of more than one layer as described in WO 03/002325 may be used for coating of a catheter with a composition of the invention. Co-extrusion represents another possibility.

In another embodiment, the part of the medical appliance to be inserted into said body cavity, as such, is made of the composition of the invention. Said part of the medical appliance, for example a catheter, may be made by injection moulding as described in WO 03/002325. Extrusion is also a possibility.

Following preparation of said medical appliance comprising a composition according to the invention, the composition is swelled in water of an aquesous solution to a degree where a slippery surface is achieved. The aqueous solution may contain an osmolality increasing water-soluble compound as described for example in EP 991702.

The aqueous solution may also contain a humectant as well as diethylene glycol, glycerol, and propylene glycol.

The coating on the medical appliances as mentioned above may be applied to the appliance using conventional techniques. The coating of the medical appliance for obtaining a slippery surface, as mentioned above, may also be in the form of a foam.

The, use of hydrogel as electroconductive compositions are described in US 4.699.146. EP 85 327 and US 4.593.053.

The adhesive composition of the invention may also be used as an electroconductive composition for attaching an electrically conductive member to a selected surface, such as mammalian tissue.

Thus, in another embodiment, the composition of the invention is an electrically conductive composition comprising a composition according to the invention as well as an aqueous solution of a salt, such as KCl, NaCl, sodium sulphate, potassium sulphate, ammonium acetate, magnesium acetate or magnesium sulphate.

The invention also relates to an electrode for establishing electrical contact with a surface comprising an electrically conductive member and an electrocoductive composition according to the invention, preferably an adhesive composition of the invention, adapted to be in contact with the skin of a living being.

The electrode is suitably a electrosurgical return electrode, a transcutaneous electrical nerve stimaultion electrode or an EKG monitoring electrode. any of claims 1-22 as well as an aqueous solution of a salt.

As mentioned, the hydrogel forming compositions according to the invention may be useful for drug delivery, and delivery of other active ingredients, including ionophoretical delivery as described in US 4.593.053.

This opens for a combined medical treatment of wounds and an easy and sterile application of the active ingredients. Incorporating active ingredients into the adhesives of the invention enables local administration of active compounds in a wound. The active ingredient may suitable be a cytochine such as growth hormone or a polypeptide growth factor in which may exercise an effect on the wound, other medicaments such as bacteriostatic or bactericidal compounds, e.g. iodine, iodopovidone complexes, chloramine, chlorohexidine, silver salts such as Alphasan 2000 or 5000 (sodiumhydrogen-silver- zirconiumphosphate), sulphadiazine, silver nitrate, silver acetate, silver lactate, silver sulphate, silver-sodium-thiosulphate, silver chloride or silver complexes, zinc or salts thereof, metronidazol, sulpha drugs, and penicillins, tissue-healing enhancing agents, e.g. RGD tripeptides and the like, proteins, amino acids such as taurine, vitamins such ascorbic acid, enzymes for cleansing of wounds, e.g. pepsin, trypsin and the like, proteinase inhibitors or metalloproteinase inhibitors such as IIIostat or ethylene diamine tetraacetic acid, cytotoxic agents and proliferation inhibitors for use in for example surgical insertion of the product in cancer tissue and/or other therapeutic agents which optionally may be used for topical application, pain relieving agents such as lidocaine, chinchocaine or non-steroid anti-inflammatory drugs (NSAIDS) such as ibuprofen, ketoprofen, fenoprofen or declofenac, emollients, retinoids or an agents having a cooling effect.

Thus, the present invention also relates to compositions of the invention, including compositions of the invention in the form of a foam, containing a pharmaceutically active ingredient, e.g. an antibacterial agent as well as dressings, including would dressings comprising such a composition.

### Description of the Preferred Embodiments

The invention is now explained more in detail in the examples below. As all suitable modifications and equivalents may be resorted to, and the examples are not to be considered as limiting the scope of the invention set forth in the appended claims.

### Example 1

### Comparative example

As a reference a water soluble hydrophilic adhesive without Amphiphilic block copolymers 30.5 grams of PVP K-90, 3.5 grams of Pemulen TR2and 31.5 grams of PEG 400 were mixed as a premix. Initially the premix of the plastizicing glycols, the PVP and the cross-linked polyacrylic acid were added and mixed in a Brabender mixer at 60 degrees C for 30 minutes for obtaining a macroscopically homogeneous mixture. The hot adhesive from the mixing chamber was moulded into a sheet of 1 mm thickness between two sheets of silicone paper.

### Example 2

### Preparation of an adhesive composition according to the invention

3.5 grams of PVP K-90, 17.5 grams of PVP K-25, 3.5 grams of Pemulen TR2 and 28 grams of PEG 400 were mixed as a premix. Initially the premix of the plastizicing glycols, the PVP and the cross-linked polyacrylic acid were added and mixed in a Brabender mixer at 100 degrees C for 10 minutes. Then 17.5 grams of the amphiphilic polyurethane (Tecogel 2000) were added slowly in order to ensure complete mixing of the components. After 20 minutes' mixing a macroscopically homogenous mixture was obtained. The hot adhesive from the mixing chamber was moulded into 1 mm thickness between two sheets of silicone paper.

### Example 3

### Preparation of an adhesive composition according to the invention

3.5 grams of PVP K-90, 17.5 grams of PVP K-25, 3.5 grams of Pemulen TR2 and 28 grams of PEG 400 were mixed as a premix. Initially the premix of the plastizicing glycols, the PVP, and the cross-linked polyacrylic acid were added and mixed in a Brabender mixer at 150 degrees C for 10 minutes. Then 17.5 grams of amphiphilic polyurethane (ESTANE T541 0) were added slowly in order to ensure complete mixing of the components. After 20 min. mixing a macroscopically homogenous mixture was obtained. The hot adhesive from the mixing chamber was moulded into 1 mm thickness between to sheets of silicone paper.

### Examples 4-9

Adhesive compositions of the invention having the compositions in grams of the constituents as described in table 2 were prepared in analogy with the procedure described in example 3.

**Table 2.**

| **Constituent** | **Ex.4** | **Ex. 5** | **Ex. 6** | **Ex. 7** | **Ex. 8** | **Ex. 9** |
|---|---|---|---|---|---|---|
| PVP K-90 | 3.5 | 3.5 | 3.5 | 0 | 0 | 0 |
| ViviPrint 540 | 0 | 0 | 0 | 3.5 | 3.5 | 3.5 |
| PVP K-12 | 17.5 | 0 | 0 | 17.5 | 0 | 0 |
| PVP K-15 | 0 | 17.5 | 0 | 0 | 17.5 | 0 |
| PVP K-30 | 0 | 0 | 17.7 | 0 | 0 | 17.5 |
| Pemulen TR-2 | 3.5 | 3.5 | 3.5 | 3.5 | 3.5 | 3.5 |
| T541 0 | 17.5 | 17.5 | 17.5 | 17.5 | 17.5 | 17.5 |
| PEG 400 | 28.0 | 28.0 | 28.0 | 28.0 | 28.0 | 28.0 |
| In total (grams) | 70 | 70 | 70 | 70 | 70 | 70 |

### Example 10

The adhesive compositions produced according to Examples were evaluated with respect to tack, release to finger, hardness, and transparency according to a scale from 0 to 5 having the significance stated in Table 3 below, The colour was also evaluated. The test methods used were to following:

### Tack

The adhesive piece (2.5*2.5 cm * 1mm) is gently pressed between the thumb and the forefinger.
1. First with dry fingers
2. Subsequently with moist fingers, dabbed on a moist sponge

If the fingers stick to the adhesive when removing them again, it is estimated as high tack - the character 5, and on the contrary if there is no tack the character given is 0.

### Release

In continuation of tack it is estimated how much adhesive is left on the forefinger and the thumb when the adhesive is removed. The fingers are gently pressed together again, and if there are a lot of residues it will be felt as high tack and estimated with a high number. On the contrary, if there are no adhesive residues on the fingers - there is no tack, and consequently a low character is given.

**Table 3.**

| **Rating 0 - 5** | | |
|---|---|---|
| Rating | 0 | 5 |
| Tack | No tack | High tack |
| Release to finger | No release | High release |

The compositions prepared in Examples 1-9 were evaluated by a skilled person according to the rating above and the ratings are stated in the below tables A1-A3.

**Table A-1**

| **Property** | **Rating** | | |
|---|---|---|---|
| | Ex. 1 | Ex. 2 | Ex. 3 |
| **Tack dry/wet** | 5/3 | 5/2 | 4/3 |
| **Release to finger dry/wet** | 2/2 | 2/2 | 1/1 |

**Table A-2**

| **Property** | **Rating** | | | | | |
|---|---|---|---|---|---|---|
| | **Ex. 4** | **Ex. 5** | **Ex. 6** | **Ex. 7** | **Ex. 8** | **Ex. 9** |
| **Tack dry/wet** | 5/3 | 5/2 | 4/3 | 5/2 | 5/3 | 3/2 |
| **Release to finger dry/wet** | 2/2 | 2/2 | 1/1 | 2/2 | 1/2 | 1/1 |

### Example 11

### Method for the preparation of an adhesive composition according to the invention

3.5 grams of PVP K-90, 17.5 grams of PVP K-25, 3.5 grams of Pemulen TR2 and 28 grams of PEG 400 is mixed as a premix. Initially the premix of the plastizicing glycols, the PVP, and the cross-linked polyacrylic acid is added and mixed in a Brabender mixer at 120 degrees C for 10 minutes. Then 17.5 grams of the amphiphilic block copolymer (Poly(styrene-b-acrylic acid-b-styrene)) is added slowly in order to ensure complete mixing of the components. After approx. 20 min. mixing a macroscopically homogenous mixture is obtained. The hot adhesive from the mixing chamber is moulded into 1 mm thickness between to sheets of silicone paper.

### Example 12

### Method for the reparation of an adhesive composition according to the invention

3.5 grams of PVP K-90, 17.5 grams of PVP K-25, 3.5 grams of Pemulen TR2, and 28 grams of PEG 400 is mixed as a premix. Initially the premix of the plastizicing glycols, the PVP, and the cross-linked polyacrylic acid is added and mixed in a Brabender mixer at 120 degrees C for 10 minutes. Then 17.5 grams of the amphiphilic block copolymer (Poly(methyl methacrylate-b-methacrylic acid-b-methyl methacrylate)) is added slowly in order to ensure complete mixing of the components. After approx. 20 min. mixing under reduced pressure a macroscopically homogenous mixture is obtained. The hot adhesive from the mixing chamber is moulded into 1 mm thickness between to sheets of silicone paper.

### Example 13

### Method for the preparation of an adhesive electroconductive composition according to the invention

3.5 grams of PVP K-90, 17.5 grams of PVP K-25, 3.5 grams of potassium chloride, and 28 grams of de-ionized water is mixed as a premix. Initially the premix of the plastizicing water and the PVP is added and mixed in a Brabender mixer at 70 degrees C for 10 minutes. Then 17.5 grams of the amphiphilic block copolymer (Poly(styrene-b-ethylene oxide-b-styrene)) is added slowly in order to ensure complete mixing of the components. After approx. 20 min. mixing to a microscopically homogenous mixture is obtained. The hot adhesive from the mixing chamber is moulded into 1 mm thickness between to sheets of silicone paper.

## Claims

1. A composition comprising one or more hydrogel-forming hydrophilic homopolymers or heteropolymers and one or more amphiphilic block-copolymers comprising hydrophobic polymer blocks being incompatible and hydrophilic polymer blocks being compatible with the hydrogel-forming hydrophilic homopolymers or heteropolymers.

2. A pressure sensitive adhesive composition comprising the composition as claimed in claim 1 wherein the hydrophilic homopolymer or heteropolymer is selected from polymers having intrinsic adhesive properties, or the composition contain a tackyfier or a plasticizer providing or improving the adhesive properties of the compostion.

3. The composition as claimed in claim 2 wherein the hydrophilic homopolymer or heteropolymer is selected from polymers having intrinsic adhesive properties.

4. The composition as claim 3 wherein the hydrophilic homopolymer or heteropolymer is a polymethylvinylether.

5. The composition as claimed in claim 2 wherein the the composition contain a tackyfier or a plasticizer providing or improving the adhesive properties of the compostion.

6. The composition according to any of claims claim 1-2 wherein the hydrophilic homopolymer or heteropolymer is poly-vinylpyrrolidone or copolymer containing vinylpyrrolidone monomer, preferably the hydrophilic homopolymer is poly-vinylpyrrolidone.

7. The composition according to any of claims 1-6 comprising a plasticizer for the hydrophilic homopolymer or heteropolymer.

8. The composition as claimed in claim 7 wherein the plasticizer is selected from the group consisting of polyethylene glycols, suitable PEG 400, and water.

9. The composition according to any of claims 1-8 wherein the hydrophobic block(s) of the amphiphilic block copolymer has a molecular weight of at least 1000, preferably between 1000 and 500.000, more preferred between 1000 and 300.000, more preferred between 1000 and 100. 000, or most preferred between 1000 and 50.000.

10. The composition according to any of claims 1-9 wherein the hydrophilic block(s) of the amphiphilic block copolymer has a molecular weight of at least 1000, preferably between 1000 and 300.000, more preferred between 50.000 and 300.000.

11. The composition according to any of claims 1-10, **characterized in that** the amphiphilic block copolymer is a triblock copolymer having the formula ABA, a diblock copolymer of formula AB, or a multi block or three or multi arm star-shaped copolymer structure, containing A and B blocks.

12. The composition according to any of claims 1-2, 9 and 11, **characterized in that** the hydrophobic block(s) of the amphiphilic block copolymer comprises polymerised styrene.

13. The composition according to claim 12 wherein the hydrophobic block(s) of the amphiphilic block copolymer consists essentially of polymerised styrene.

14. The composition according to any of claims 1-2, 9 and 11, **characterized in that** the hydrophobic block(s) of the amphiphilic block copolymer comprises polymerised hydrophobic (meth)acrylic ester.

15. The composition according to claim 14 wherein the hydrophobic block(s) consists essentially of polymerised (meth)acrylic ester.

16. The composition according to any of claims 1-2, 9 and 11, **characterized in that** the hydrophobic block(s) of the amphiphilic block copolymer comprises polymerised vinylic unsaturated aliphatic hydrocarbon comprising from 1 to 6 carbon atoms.

17. The composition according to claim 16, **characterized in that** the vinylic unsaturated hydrocarbon comprises 4 carbon atoms

18. The composition according to claims 1-2, 9 and 11-15 wherein the amphiphilic block copolymer comprises at least two hydrophobic blocks, one of the hydrophobic block(s) consisting essentially of polymerised styrene and another hydrophobic block consisting essentially of polymerised (meth)acrylic acid ester.

19. The composition according to any of claims 1-2 and 10-11, **characterized in that** the hydrophilic block(s) of the amphiphilic block copolymer comprises polymerised monomers selected from ethylenically unsaturated monocarboxylic and dicarboxylic acid monomers, such as acrylic acid, methacrylic acid, itaconic acid, maleic acid and fumaric acid; and monoalkyl esters of dicarboxylic acids and their N-substituted derivatives (amides), amides of unsaturated carboxylic acids, such as acrylamide, methacrylamide, N-methoxyacrylamide, acrylamide or methacrylamide, and N-alkylacrylamides; ethylenic monomers containing a sulphonic acid group and ammonium or alkali metal salts thereof, amides of vinylamine and unsaturated ethylenic monomers containing a secondary, tertiary or quaternary amino group, or a heterocyclic group containing nitrogen, and aminoalkyl (meth)acrylamides and zwitterionic monomers.

20. The composition according to claim 19, **characterized in that** the hydrophilic block(s) of the amphiphilic block copolymer comprises polymerised acrylic acid and salts thereof.

21. The composition according to any of claims 1-2,10-11 wherein the hydrophilic block is a polyethylene glycol or a homopolymer or copolymer of acrylic acid, maleic acid, hydroxyethylmethacrylate (HEMA), vinylpyrrolidone (NVP), polyethyleneglycol(meth)acrylate, ethoxypolyethyleneglycol(meth)acrylate, methoxyethyl(meth)acrylate, ethoxy(meth) acrylate, 2-dimethylaminoethyl (meth)acrylate (DMAEMA) and 3-dimethylaminopropylmethacrylamid (DMAPMA).

22. The composition according to any of claims 1-2 **characterized in that** the amphiphilic block copolymer is an amphiphilic polyurethane block copolymer.

23. The composition according to any of claims 1-22 in the form of foam.

24. A composition according to any of claims 1-23 containing a pharmaceutically active compound, such as an antibacterial agent.

25. A dressing, e.g. a wound dressing where the skin facing surface and/or the body of the dressing comprises a composition according to any of claims 1-24.

26. A medical device adapted for being attached to the surface of a living being comprising a pressure sensitive adhesive composition according to any of claims 1-23 at the surface, which is to be attached to the surface of the living being.

27. Use of an adhesive composition according to any of claims 1-23 for securing items, such as wound dressings, wound drainage bandages, ostomy, or protheses to the skin.

28. Use of an adhesive composition according to any of claims 1-23 for sealing around an ostomy.

29. A medical appliance, e.g. a surgical suture, a catheter or a guidewire, for introduction into a natural or artificial body cavity of a living being, where at least the part of said appliance to be inserted into said body cavity comprises a composition according to claims 1-23.

30. The medical appliance according to claim 29, which is carrying said composition as a coating at least on its outer surface.

31. The medical appliance according to claim 29, where at least the part of the medical appliance to be inserted into said body cavity is made of said composition.

32. The medical appliance according to any of claims 29-31, wherein said composition contains water or an aqueous solution in an amount sufficient to achieve a slippery surface of the composition.

33. The medical appliance according to claim 32 wherein the aqueous solution contain an osmolality increasing water-soluble compound.

34. The medical appliance according to claim 31 wherein said aqueous solution contain glycerol.

35. An electrically conductive composition comprising a composition according to any of claims 1-22 as well as an aqueous solution of a salt.

36. An electrode comprising an electrically conductive member and an electrocoductive composition according to 35 being adapted to be in contact with the surface of a living being.
